# EUROPEAN PATENT SPECIFICATION

(11) **EP 2 134 381 B1**
(45) Date of publication and mention of the grant of the patent: **31.12.2014**
(21) Application number: 08743777.8
(22) Date of filing: 11.03.2008
(51) Int. Cl.: A61L 31/06, A61L 31/14

(54) **IMPLANTABLE MEDICAL DEVICES FABRICATED FROM BLOCK COPOLYMERS**
IMPLANTIERBARE MEDIZINISCHE VORRICHTUNGEN AUS BLOCKCOPOLYMEREN
DISPOSITIFS MÉDICAUX IMPLANTABLES FABRIQUÉS À PARTIR DE COPOLYMÈRES SÉQUENCÉS

(30) Priority: 28.03.2007 US 729506
(43) Date of publication of application: 23.12.2009
(73) Proprietor: Abbott Cardiovascular Systems Inc., Santa Clara, CA 95054-2807 (US)
(72) Inventor: WANG, Yunbing, Sunnyvale, California 94807 (US); GALE, David C., Kennesaw, GA 30152 (US)
(74) Representative: ZBM Patents - Zea, Barlocci & Markvardsen
(86) International application number: PCT/US2008/056539
(87) International publication number: WO 2008/121508

(56) References cited:
- WO-A1-99/02168
- WO-A1-02/096967
- WO-A1-2008/124312
- WO-A2-2006/049907
- WO-A2-2007/143116
- WO-A2-2009/045808
- ASPLUND BASSE; ET AL: "Effects of hydrolysis on a new biodegradable co-polymer" JOURNAL OF BIOMATERIALS SCIENCE. POLYMER EDITION, VSP, UTRECHT, vol. 17, 1 January 2006 (2006-01-01), pages 615-630, XP008092308 ISSN: 0920-5063

## Description

### BACKGROUND OF THE INVENTION

### Field of the Invention

This invention relates to implantable medical devices fabricated from block copolymers.

### Description of the State of the Art

This invention relates to radially expandable endoprostheses, which are adapted to be implanted in a bodily lumen. An "endoprosthesis" corresponds to an artificial device that is placed inside the body. A "lumen" refers to a cavity of a tubular organ such as a blood vessel.

A stent is an example of such an endoprosthesis. Stents are generally cylindrically shaped devices, which function to hold open and sometimes expand a segment of a blood vessel or other anatomical lumen such as urinary tracts and bile ducts. Stents are often used in the treatment of atherosclerotic stenosis in blood vessels. "Stenosis" refers to a narrowing or constriction of the diameter of a bodily passage or orifice. In such treatments, stents reinforce body vessels and prevent restenosis following angioplasty in the vascular system. "Restenosis" refers to the reoccurrence of stenosis in a blood vessel or heart valve after it has been treated (as by balloon angioplasty, stenting, or valvuloplasty) with apparent success.

The treatment of a diseased site or lesion with a stent involves both delivery and deployment of the stent. "Delivery" refers to introducing and transporting the stent through a bodily lumen to a region, such as a lesion, in a vessel that requires treatment. "Deployment" corresponds to the expanding of the stent within the lumen at the treatment region. Delivery and deployment of a stent are accomplished by positioning the stent about one end of a catheter, inserting the end of the catheter through the skin into a bodily lumen, advancing the catheter in the bodily lumen to a desired treatment location, expanding the stent at the treatment location, and removing the catheter from the lumen.

In the case of a balloon expandable stent, the stent is mounted about a balloon disposed on the catheter. Mounting the stent typically involves compressing or crimping the stent onto the balloon. The stent is then expanded by inflating the balloon. The balloon may then be deflated and the catheter withdrawn. In the case of a self-expanding stent, the stent may be secured to the catheter via a constraining member such as a retractable sheath or a sock. When the stent is in a desired bodily location, the sheath may be withdrawn which allows the stent to self-expand.

The stent must be able to satisfy a number of mechanical requirements. First, the stent must be capable of withstanding the structural loads, namely radial compressive forces, imposed on the stent as it supports the walls of a vessel. Therefore, a stent must possess adequate radial strength. Radial strength, which is the ability of a stent to resist radial compressive forces, is due to strength and rigidity around a circumferential direction of the stent. Radial strength and rigidity, therefore, may also be described as, hoop or circumferential strength and rigidity.

Once expanded, the stent must adequately maintain its size and shape throughout its service life despite the various forces that may come to bear on it, including the cyclic loading induced by the beating heart. For example, a radially directed force may tend to cause a stent to recoil inward. Generally, it is desirable to minimize recoil. In addition, the stent must possess sufficient flexibility to allow for crimping, expansion, and cyclic loading. Longitudinal flexibility is important to allow the stent to be maneuvered through a tortuous vascular path and to enable it to conform to a deployment site that may not be linear or may be subject to flexure. Finally, the stent must be biocompatible so as not to trigger any adverse vascular responses.

The structure of a stent is typically composed of scaffolding that includes a pattern or network of interconnecting structural elements often referred to in the art as struts or bar arms. The scaffolding can be formed from wires, tubes, or sheets of material rolled into a cylindrical shape. The scaffolding is designed so that the stent can be radially compressed (to allow crimping) and radially expanded (to allow deployment). A conventional stent is allowed to expand and contract through movement of individual structural elements of a pattern with respect to each other.

Additionally, a medicated stent may be fabricated by coating the surface of either a metallic or polymeric scaffolding with a polymeric carrier that includes an active or bioactive agent or drug. Polymeric scaffolding may also serve as a carrier of an active agent or drug.

Furthermore, it may be desirable for a stent to be biodegradable. In many treatment applications, the presence of a stent in a body may be necessary for a limited period of time until its intended function of, for example, maintaining vascular patency and/or drug delivery is accomplished. Therefore, stents fabricated from biodegradable, bioabsorbable, and/or bioerodable materials such as bioabsorbable polymers should be configured to completely erode only after the clinical need for them has end.

WO02/096967 A1 discloses a non-biodegradable article of manufacture comprising an implantable prosthesus or implant formed of an arborescent branched block copolymer of a polyisoolefin and a polymonovinylidene arene characterized by having thermoplastic elastomeric properties wherein the aforesaid arborescent branched block copolymer comprises an arborescent branched polyisoolefin polymer block and some of the branches of the aforesaid arborescent branched polyisoolefin block terminate in polymonovinylidene arene plastic endblock.

Asplund Basse *et al.* (Asplund Basse et al., "Effects of hydrolysis on a new biodegradable co-polymer", Journal of Biomaterials Science. Polymer Edition, VSP, Utrecht, vol. 17, 01 January 2006) discloses a thermoplastic elastomer consisting of PLLA, PTMC and PCL. Said elastomer is applied in the form of a film on a stent.

Potential problems with polymeric implantable medical devices, such as stents, include insufficient toughness and slow degradation rate.

### SUMMARY OF THE INVENTION

Various embodiments of the present invention include an implantable medical device comprising a structural element, the structural element fabricated from a polymeric material including a block copolymer having a continuous phase block that is immiscible with a discrete phase block, the structural element comprising a plurality of discrete phase regions dispersed within a continuous phase, wherein a majority of the continuous phase comprises continuous phase blocks of the block copolymer, the discrete phase regions comprising discrete phase blocks of the block copolymer.

Certain embodiments of the present invention include a stent comprising a structural element, the structural element fabricated from a polymeric material including a block copolymer having glassy blocks and elastic blocks, wherein the elastic blocks form elastic discrete phase regions and the glassy blocks form a glassy continuous phase, the elastic discrete phase regions being dispersed within the glassy continuous phase.

### BRIEF DESCRIPTION OF THE DRAWINGS

FIG. 1A depicts a view of a stent.
FIG. 1B depicts a section of a structural element from the stent depicted in FIG. 1A.
FIG. 2 depicts a schematic close-up view of the section depicted in FIG. 1B.
FIGs. 3A-E depict several embodiments of block copolymers.
FIG. 4 depicts a schematic close-up view of a discrete polymer phase dispersed within a continuous polymer phase.

### DETAILED DESCRIPTION OF THE INVENTION

Various embodiments of the present invention include an implantable medical device fabricated from block copolymers having continuous phase blocks that are immiscible with discrete phase blocks. The discrete phase blocks form discrete phase regions dispersed within a continuous phase composed of continuous phase blocks.

As used herein, an "implantable medical device" includes, but is not limited to, self-expandable stents, balloon-expandable stents, stent-grafts, other expandable tubular devices for various bodily lumen or orifices, implantable cardiac pacemakers and defibrillators, leads and electrodes for the preceding, vascular grafts, grafts, artificial heart valves, and cerebrospinal fluid shunts. An implantable medical device can be designed for the localized delivery of a therapeutic agent. A medicated implantable medical device may be constructed by coating the device or substrate with a coating material containing a therapeutic agent. The substrate of the device may also contain a therapeutic agent.

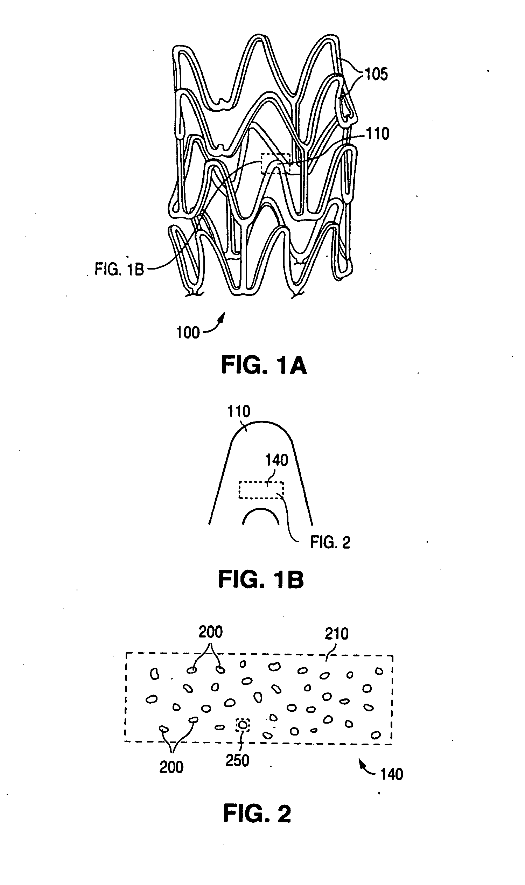

FIG. 1A depicts a view of a stent 100. In some embodiments, a stent may include a pattern or network of interconnecting structural elements 105. Stent 100 may be formed from a tube (not shown). The pattern of structural elements 105 can take on a variety of patterns. The structural pattern of the device can be of virtually any design. The embodiments disclosed herein are not limited to stents or to the stent pattern illustrated in FIG. 1A. The embodiments are easily applicable to other patterns and other devices. The variations in the structure of patterns are virtually unlimited. A stent such as stent 100 may be fabricated from a tube by forming a pattern with a technique such as laser cutting or chemical etching.

An implantable medical device is made completely from a biodegradable, bioabsorbable, biostable polymer, or a combination thereof. The terms biodegradable, bioabsorbable, and bioerodable are used interchangeably and refer to polymers that are capable of being completely degraded and/or eroded when exposed to bodily fluids such as blood and can be gradually resorbed, absorbed, and/or eliminated by the body. The processes of breaking down and absorption of the polymer can be caused by, for example, hydrolysis and metabolic processes.

The embodiments of the present invention address certain shortcomings of biodegradable polymers as they relate to their application to implantable medical devices. For example, some biodegradable polymers have a degradation rate that is slower than desired for certain stent treatments. As a result, the degradation time of a stent made from such polymers can be longer than desired. For example, a stent made from a semicrystalline polymer such as PLLA can have a degradation time between about two and three years. In some treatment situations, a shorter degradation time is desirable, for example, less than a year.

An additional shortcoming of some polymers is that their toughness can be lower than desired, in particular, for use in stent applications. For example, polymers such as PLLA tend to be brittle under physiological conditions. Physiological conditions refer to conditions to which an implant is exposed to within a human body. Physiological conditions include, but are not limited to, human body temperature, approximately 37 °C. Specifically, certain semicrystalline polymers such as PLLA can have a Tg above human body temperature. These polymers can exhibit a brittle fracture mechanism at these conditions in which there is little or no plastic deformation prior to failure. As a result, a stent fabricated from such polymers can have insufficient toughness for the range of use of a stent.

In general, one way to increase fracture toughness of a polymer is to incorporate a discrete phase having a higher or relatively high fracture toughness within the polymer. The discrete phase can be another polymer that is immiscible with the original polymer. For example, the discrete phase can be a rubbery polymer or elastomeric polymer. With respect to implantable medical device applications, the discrete phase should have a higher fracture toughness as physiological conditions. The fracture toughness of a device is increased since the discrete phase can absorb energy arising from stress imparted to a device. To ensure good energy transfer between interfaces of the phases, it is important that there be sufficient bonding or adhesion between the phases. See, Y. Wang, etc. Journal of Polymer Science Part A: Polymer Chemistry, 39, 2001, 2755-2766.

Embodiments of the present invention reduce or eliminate the shortcomings of biodegradable polymers discussed above. Various embodiments can include chemical modification of polymers, such as glassy, semicrystalline polymers, that increase degradation rate, increase fracture toughness, or both. "Glassy" refers to a polymer that exhibits a brittle fracture mechanism. In some embodiments, such polymers can be modified by adding blocks or segments to the original polymer that are immiscible with the original polymer. The added blocks form discrete phase regions within a continuous phase composed of the original polymer segments. The added blocks can be faster eroding, tougher, or both than the original polymer. As a result the modified polymer has a higher degradation rate, is tougher, or both than the unmodified polymer.

The present invention in directed to an implantable medical device fabricated from a polymeric material including a block copolymer having a continuous phase block that is immiscible with a discrete phase block. The structural element includes a plurality of discrete phase regions dispersed within a continuous phase. A majority of the continuous phase includes or is composed of continuous phase blocks of the block copolymer. The discrete phase regions are composed of discrete phase blocks of the block copolymer. The continuous phase blocks are substantially longer than discrete phase blocks. In certain embodiments, the ratio of the molecular weight of the continuous phase blocks to discrete phase blocks is between about 2:1 to 10:1.

In some embodiments, the continuous phase has a high rigidity and a relatively low fracture toughness at physiological conditions. High rigidity and strength are important, for example, for stent applications so that a stent can support the walls of a vessel. The discrete phase can have a higher fracture toughness than the continuous phase at physiological conditions. The discrete phase tends to increase the toughness of the polymeric material, and thus, the structural element. The adhesion between the continuous and discrete phases is enhanced by the chemically bonding between the continuous phase blocks and discrete phase blocks. The high degree of adhesion provided by the chemical bonding facilitates energy transfer between the phases.

FIG. 1B depicts a section of a segment 110 of strut 105 from the stent depicted in FIG. 1A. FIG. 2 depicts a microscopic schematic view of a portion 140 of segment 110 of a strut as depicted in FIG. 1B. Portion 140 includes a plurality of discrete phase regions 200 dispersed within a continuous phase 210.

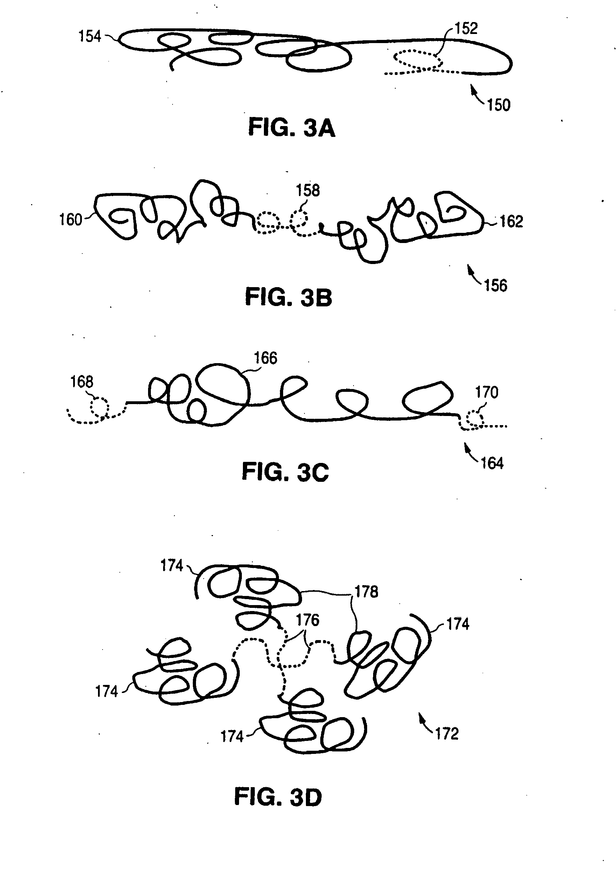

There are several ways of modifying a glassy, semicrystalline polymer with the added blocks as described herein. FIGs. 3A-E depict several embodiments of block copolymers in which a polymer can be modified to include discrete phase blocks. FIG. 3A depicts a di-block copolymer 150 in which a first end has a discrete phase block 152 (shown as a broken line) and a second end has a continuous phase block 154 (shown as a solid line). FIG. 3B depicts a tri-block copolymer 156 that has a discrete phase block 158 between continuous phase blocks 160 and 162. FIG. 3C depicts a tri-block copolymer 164 with a continuous phase block 166 between discrete phase blocks 168 and 170.

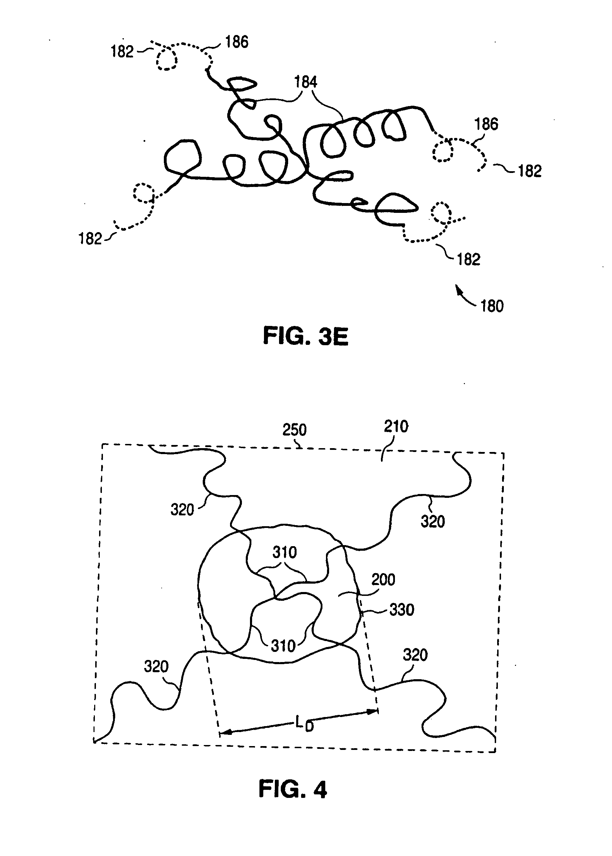

In other embodiments, the block copolymer can be a star-block copolymer having at least three arms. FIG. 3D depicts a star-block copolymer 172 having four arms 174. Arms 174 have inner segments 176 (shown as broken lines) and outer segments 178. Inner segments 176 are discrete phase blocks and outer segments 178 are continuous phase blocks. FIG. 3E depicts a star-block copolymer 180 having four arms 182. Arms 182 have inner segments 184 and outer segments 186. Inner segments 184 are continuous phase blocks and outer segments 186 are discrete phase blocks.

FIG. 4 depicts a schematic close-up view of section 250 of FIG. 2 of a discrete phase region 200 and the interface between discrete phase region 200 and continuous phase 210. Section 250 includes a star-block copolymer of FIG. 3D with inner segments 310 and outer segments 320. Line 330 delineates an approximate boundary between discrete phase region 200 and continuous phase 210. L_{D} is a characteristic dimension of discrete phase region 200.

It is believed that when a device is placed under stress, the discrete phase tends to absorb energy when a fracture starts to propagate through a structural element. Crack propagation through the continuous phase may then be reduced or inhibited. As a result, fracture toughness of the polymeric material, and thus, the structural element tends to be increased. The discrete phase regions are anchored within the continuous phase through chemical bonding, increasing the adhesion between the discrete phase and the continuous phase. It is believed that without the anchoring or adhesion provided by the chemical bonding, a propagating crack may go around the discrete phase regions, reducing the effectiveness of the discrete phase in absorbing energy imparted to a device.

Generally, it is desirable for the discrete phase regions to be uniformly or substantially uniformly dispersed throughout the continuous polymer phase to facilitate the increase in toughness. The more dispersed the discrete phase regions, the greater is the increase in toughness. Additionally, the increase in toughness is related to the size of the discrete phase. Both the degree of dispersion and discrete phase size can be controlled by the length or molecular weight of the discrete phase blocks. The characteristic length of a discrete phase can be 1 nm to 100 nm, 100 nm to 500 nm, 500 nm to 1,000 nm, 1000 nm to 10,000 nm, or greater than 10,000 nm. The molecular weight of the discrete phase blocks is between 1 kg/mol to 50 kg/mol to obtain a desired characteristic length of the discrete phase regions while the molecular weight of the continuous phase block is between 50 kg/mol to 1000 kg/mol.

In some embodiments, the discrete phase blocks of the block copolymer include units that form polymers having a higher fracture toughness at physiological conditions than a glassy, semicrystalline polymer, such as PLLA. The discrete phase blocks can form a discrete phase that is more flexible and has a lower modulus than the continuous phase blocks of the continuous phase. The continuous phase blocks can be selected to have a Tg above body temperature, so that the device remains rigid after implantation. Generally, discrete phase blocks may be selected that have a Tg below body temperature. In one embodiment, the discrete phase blocks of the block polymer can be a rubbery or elastomeric polymer. An "elastomeric" or "rubbery" polymer refers to a polymer that exhibits elastic deformation though all or most of a range of deformation. In some embodiments, the discrete phase can be substantially or completely amorphous.

Biodegradable polymers having a relatively high fracture toughness at body temperature include, but are not limited to, polycaprolactone (PCL), poly(tetramethyl carbonate) (PTMC), and polydioxanone. Some embodiments of the discrete phase blocks of the block polymer can include caprolactone (CL), tetramethyl carbonate (TMC), dioxanone units, or a combination thereof.

Furthermore, a device fabricated from embodiments of the polymeric material can address issues relating to the degradation rate of polymer implantable medical devices. As indicated above, a glassy, semicrystalline polymer, such as PLLA, can have a degradation rate that is slower than desired for certain stent treatments. The slow degradation rate is due at least in part to the crystallinity of the glassy polymer. In some embodiments, the discrete phase blocks of the block copolymer can be faster degrading than the continuous phase blocks. The faster degradation can be due at least in part to the amorphous structure of the discrete phase since the diffusion rate of fluids through an amorphous structure is generally faster than through a crystalline structure. The faster degrading discrete phase regions increase water penetration and content in the discrete phase and in the continuous phase. The increased water penetration and content causes an increase in the degradation rate of the polymeric material, and thus, the device.

In additional embodiments, the block copolymer can include units in the discrete phase blocks with characteristics that tend to increase the degradation rate of the polymeric material. For example, the discrete phase blocks can include units that are more hydrophilic than the units in the continuous phase blocks. The discrete phase blocks can also have units that are more hydrolytically active than the continuous phase blocks. These two characteristics increase the moisture content of the polymeric material which increases the degradation rate. Additionally, the discrete phase blocks can also have units that have acidic and hydrophilic degradation products. Since the rate of the hydrolysis reaction tends to increase as the pH decreases, acidic degradation products can increase the degradation rate of the polymeric material and the device. Glycolide (GA) units, for example, have acidic degradation products which can increase the degradation rate of a polymeric material when included in a discrete block.

In some embodiments, the discrete phase blocks can include units that increase the fracture toughness (toughness-enhancing units) of the polymeric material and units that have one or more of the characteristics that increase degradation rate mentioned above (fast degrading units). In exemplary embodiments, the discrete phase blocks can include GA units and CL or TMC units. In other exemplary embodiments, the discrete phase blocks can include GA, CL, and TMC units. For example, the discrete phase blocks can be P(GA-co-CL) or P(GA-co-TMC), or P(GA-co-TMC-co-CL). These discrete phase blocks can have alternating or random GA, CL, and TMC units.

In some embodiments, the flexibility and degradation rate of the discrete phase blocks can be adjusted by the ratio of fast degrading and toughness-enhancing units. As the ratio of CL, for example, increases in P(GA-co-CL), the block copolymer becomes more flexible and tougher. The Tg of the discrete phase blocks can be tuned to a desired value by adjusting the ratio of component monomers. For example, the Tg of the discrete phase may be engineered to be less than a body temperature to provide a more flexible discrete phase under physiological conditions. Additionally, the degradation rate of the discrete phase blocks, and thus the polymeric material, can be increased by increasing the fraction of GA in the discrete phase blocks. In exemplary embodiments, the P(GA-co-CL) or P(GA-co-TMC) segments can have greater than 1 wt%, 5 wt%, 20 wt%, 50 wt%, 70 wt%, or 80 wt% GA units.

In exemplary embodiments, a block copolymer of the implantable medical device can have PLLA continuous phase blocks. The PLLA blocks can be chemically bonded with discrete phase blocks including, but not limited to, P(GA-co-CL), P(GA-co-TMC), P(GA-co-CL-co-TMC), or P(CL-co-TMC). In exemplary embodiments, the polymeric material can include 1-30 wt%, or more narrowly, 2-20 wt% of the discrete phase blocks and about 80-98 wt% of the continuous phase blocks. The chemical bonding between the PLLA blocks and the discrete phase blocks binds the discrete phase with the continuous phase, facilitating the increase in the fracture toughness.

Exemplary diblock copolymers include PLLA-b-P(CL-co-TMC), PLLA-b-P(GA-co-TMC), PLLA-b-P(GA-co-CL), and PLLA-b-P(GA-co-CL-co-TMC), long continuous phase PLLA blocks being bonded to a shorter discrete phase block in the middle.

Exemplary triblock copolymers include PLLA-b-P(CL-co-TMC)-b-PLLA, PLLA-b-P(GA-co-TMC)-b-PLLA, PLLA-b-P(GA-co-CL)-b-PLLA, and PLLA-b-P(GA-co-CL-co-TMC)-b-PLLA, long discrete phase PLLA blocks at two ends and short discrete phase blocks in the middle.

Other exemplary triblock copolymers include P(CL-co-TMC)-b-PLLA-b-P(CL-co-TMC), P(GA-co-TMC)-b-PLLA-b-P(GA-co-TMC), (GA-co-CL)-b-PLLA-b-P(GA-co-CL), and (GA-co-TMC-co-CL)-b-PLLA-b-P(GA-co-TMC-co-CL), long continuous phase PLLA blocks in the middle and short discrete phase blocks at two ends.

Exemplary star-block copolymers include PLLA-b-P(CL-co-TMC), PLLA-b-P(GA-co-TMC), PLLA-b-P(GA-co-CL), and PLLA-b-P(GA-co-CL-co-TMC) with short, inner core discrete phase blocks and long continuous phase PLLA blocks as an outer shell. Alternatively, the star-block copolymer can have long continuous phase PLLA blocks as an inner core and short discrete phase blocks as an outer shell.

In some embodiments, the block copolymers described herein can be formed by solution-based polymerization. Other methods can be used, such as, without limitation, melt phase polymerization. In solution-based polymerization, all the reactive components involved in the polymerization reaction are dissolved in a solvent.

In embodiments of solution polymerization, monomer units of the blocks, an initiator, a catalyst, and a solvent are used. The first step generally involves forming a precursor block, either the discrete phase block or the continuous phase block. Monomer units of the precursor block, a suitable initiator, and a suitable catalyst are added to a suitable solvent to form a polymerization solution. After the formation of the precursor block, monomer units of the second block and catalyst are then added to the solution to form the block copolymer with discrete phase blocks and continuous phase blocks. The solvent(s) for forming the second blocks can be selected so that the precursor block is soluble in the solvent(s) to enable the precursor blocks to copolymerize with the added units of the second block.

In particular, the discrete phase segments are formed first in the synthesis of a diblock copolymer, a triblock copolymer with continuous phase blocks at each end and a discrete phase block in the middle, and a star-block copolymer with an inner core of discrete phase segments. In addition, the continuous phase blocks are formed first in the synthesis of a triblock copolymer with discrete phase blocks at each end and a continuous phase block in the middle, and a star-block copolymer with an inner core of continuous phase segments.

The polymeric material may be formed into a polymer construct, such as a tube or sheet which can be rolled or bonded to form a construct such as a tube. An implantable medical device may then be fabricated from the construct. For example, a stent can be fabricated from a tube by laser machining a pattern in to the tube. In another embodiment, a polymer construct may be formed from the polymeric material using an injection molding apparatus.

In general, representative examples of polymers that may be used to fabricate an implantable medical device as described herein include, but are not limited to, poly(N-acetylglucosamine) (Chitin), Chitosan, poly(hydroxyvalerate), poly(lactide-co-glycolide), poly(hydroxybutyrate), poly(hydroxybutyrate-co-valerate), polyorthoester, polyanhydride, poly(glycolic acid), poly(glycolide), poly(L-lactic acid), poly(L-lactide), poly(D,L-lactic acid), poly(L-lactide-co-glycolide); poly(D,L-lactide), poly(caprolactone), poly(trimethylene carbonate), polyethylene amide, polyethylene acrylate, poly(glycolic acid-co-trimethylene carbonate), co-poly(ether-esters) (e.g. PEO/PLA), polyphosphazenes, biomolecules (such as fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (such as polyvinyl chloride), polyvinyl ethers (such as polyvinyl methyl ether), polyvinylidene halides (such as polyvinylidene chloride), polyacrylonitrile, polyvinyl ketones, polyvinyl aromatics (such as polystyrene), polyvinyl esters (such as polyvinyl acetate), acrylonitrile-styrene copolymers, ABS resins, polyamides (such as Nylon 66 and polycaprolactam), polycarbonates, polyoxymethylenes, polyimides, polyethers, polyurethanes, rayon, rayon-triacetate, cellulose, cellulose acetate, cellulose butyrate, cellulose acetate butyrate, cellophane, cellulose nitrate, cellulose propionate, cellulose ethers, and carboxymethyl cellulose.

Additional representative examples of polymers that may be especially well suited for use in fabricating an implantable medical device according to the methods disclosed herein include ethylene vinyl alcohol copolymer (commonly known by the generic name EVOH or by the trade name EVAL), poly(butyl methacrylate), poly(vinylidene fluoride-co-hexafluororpropene) (e.g., SOLEF 21508, available from Solvay Solexis PVDF, Thorofare, NJ), polyvinylidene fluoride (otherwise known as KYNAR, available from ATOFINA Chemicals, Philadelphia, PA), ethylene-vinyl acetate copolymers, and polyethylene glycol.

For the purposes of the present invention, the following terms and definitions apply:
For the purposes of the present invention, the following terms and definitions apply:
   The "glass transition temperature," Tg, is the temperature at which the amorphous domains of a polymer change from a brittle vitreous state to a solid deformable or ductile state at atmospheric pressure. In other words, the Tg corresponds to the temperature where the onset of segmental motion in the chains of the polymer occurs. When an amorphous or semicrystalline polymer is exposed to an increasing temperature, the coefficient of expansion and the heat capacity of the polymer both increase as the temperature is raised, indicating increased molecular motion. As the temperature is raised the actual molecular volume in the sample remains constant, and so a higher coefficient of expansion points to an increase in free volume associated with the system and therefore increased freedom for the molecules to move. The increasing heat capacity corresponds to an increase in heat dissipation through movement. Tg of a given polymer can be dependent on the heating rate and can be influenced by the thermal history of the polymer. Furthermore, the chemical structure of the polymer heavily influences the glass transition by affecting mobility.
   "Stress" refers to force per unit area, as in the force acting through a small area within a plane. Stress can be divided into components, normal and parallel to the plane, called normal stress and shear stress, respectively. True stress denotes the stress where force and area are measured at the same time. Conventional stress, as applied to tension and compression tests, is force divided by the original gauge length.
   "Strength" refers to the maximum stress along an axis which a material will withstand prior to fracture. The ultimate strength is calculated from the maximum load applied during the test divided by the original cross-sectional area.
   "Modulus" may be defined as the ratio of a component of stress or force per unit area applied to a material divided by the strain along an axis of applied force that results from the applied force. For example, a material has both a tensile and a compressive modulus. A material with a relatively high modulus tends to be stiff or rigid. Conversely, a material with a relatively low modulus tends to be flexible. The modulus of a material depends on the molecular composition and structure, temperature of the material, amount of deformation, and the strain rate or rate of deformation. For example, below its Tg, a polymer tends to be brittle with a high modulus. As the temperature of a polymer is increased from below to above its Tg, its modulus decreases.
   "Strain" refers to the amount of elongation or compression that occurs in a material at a given stress or load.
   "Elongation" may be defined as the increase in length in a material which occurs when subjected to stress. It is typically expressed as a percentage of the original length.
   "Toughness" is the amount of energy absorbed prior to fracture, or equivalently, the amount of work required to fracture a material. One measure of toughness is the area under a stress-strain curve from zero strain to the strain at fracture. Thus, a brittle material tends to have a relatively low toughness.
   "Solvent" is defined as a substance capable of dissolving or dispersing one or more other substances or capable of at least partially dissolving or dispersing the substance(s) to form a uniformly dispersed solution at the molecular- or ionic-size level at a selected temperature and pressure. The solvent should be capable of dissolving at least 0.1 mg of the polymer in 1 ml of the solvent, and more narrowly 0.5 mg in 1 ml at the selected temperature and pressure, for example, ambient temperature and ambient pressure.

### Examples

The examples set forth below are for illustrative purposes only and are in no way meant to limit the invention. The following prophetic examples are given to aid in understanding the invention, but it is to be understood that the invention is not limited to the particular materials or procedures of examples.

Examples 1, 2a-b, and 3a-b illustrate the synthesis of various block copolymers with a longer continuous phase PLLA block and shorter discrete phase fast eroding, elastic block.

### Example 1

### Synthesis of PLLA-b-P(GA-co-TMC) diblock copolymer with long PLLA block and short P(GA-co-TMC) block

In this synthesis scheme, dodecanol as initiator, GA, TMC, and L-lactide (LLA) as monomers; stannous octoate as catalyst; and xylene as solvent are used.
Step 1: 20g GA, 20g TMC, 372 mg dodecanol and 80mL xylene are added into a reactor free of moisture and oxygen to form a polymerization solution. All chemicals are mixed through mechanical stirring and the solution is heated to 90° C. Then, 140 mg stannous octoate is added into the polymerization solution and the solution is stirred at 120° C for 72 h to form a short P(GA-co-TMC) block.
Step 2: 200g LLA, 30mL xylene, and 562mg stannous octoate are then added into the reactor to form a long PLLA block.
Step 3: Approximately 48 h later, the final product is precipitated into methanol, and dried in a vacuum oven at 95° C for 48 h or till constant weight.

### Example 2a

### Synthesis of PLLA-b-P(GA-co-TMC)-b-PLLA triblock copolymer with long PLLA block at two ends and short P(GA-co-TMC) block in the middle

In this synthesis scheme, ethylene glycol as initiator; GA, TMC, and LLA as monomers; stannous octoate as catalyst; and xylene as solvent are used.
Step 1: 20g GA, 20g TMC, 124mg ethylene glycol and 80mL xylene are added into a reactor free of moisture and oxygen to form a polymerization solution. All chemicals are mixed through mechanical stirring and the solution is heated to 90° C. Then 140mg stannous octoate is added into the polymerization solution and the solution is stirred at 120° C for 72 h to form a short P(GA-co-TMC) block.
Step 2: 200g LLA, 300mL xylene, and 562mg stannous octoate are added into the reactor to form a long PLLA block at two ends.
Step 3: Approximately 48 h later, the final product is precipitated into methanol, and dried in a vacuum oven at 95° C for 48 h or till constant weight.

### Example 2b

### Synthesis of P(GA-co-TMC)-b-PLLA-b-p(GA-co-TMC) triblock copolymer with long PLLA block in the middle and short P(GA-co-TMC) block at two ends

Step 1: 200g LLA, 124mg ethylene glycol, and 300mL xylene are added into a reactor free of moisture and oxygen to form a polymerization solution. All chemicals are mixed through mechanical stirring and the solution is heated to 90° C. Then 562mg stannous octoate is added into the polymerization solution and the solution is stirred at 120° C for 48 h to form a long PLLA block.
Step 2: 20g GA, 20g CL, 80mL xylene, and 140mg stannous octoate are added into the reactor to form short P(GA-co-TMC) block at two ends.
Step 3: Approximately 72 h later, the final product is precipitated into methanol, and dried in a vacuum oven at 95° C for 48 h or till constant weight.

### Example 3a

### Synthesis of P(GA-co-TMC)-b-PLLA star-copolymer with short P(GA-co-TMC) inner core and long PLLA outer shell

In this synthesis scheme, pentaerythritol as initiator; GA, TMC, and LLA as monomers; stannous octoate as catalyst; and xylene as solvent are used.
Step 1: 20g GA, 20g TMC, 136mg pentaerythritol, 80mL xylene are added into a reactor free of moisture and oxygen to form a polymerization solution. All chemicals are mixed through mechanical stirring and the solution is heated to 90° C. Then 140 mg stannous octoate is added into the polymerization solution and the solution is stirred at 120° C for 72 h to form short P(GA-co-TMC) blocks.
Step 2: 200g LLA, 300 mL xylene, and 562mg stannous octoate are then added into the reactor to form long PLLA blocks.
Step 3: Approximately 48 h later, the final product is precipitated into methanol, and dried in a vacuum oven at 95° C for 48 h or till constant weight.

### Example 3b

### Synthesis of PLLA-b-p(GA-co-TMC) star-copolymer with long PLLA inner core and short P(GA-co-CL) outer shell

Step 1: 200g LLA, 136 mg pentaerythritol, and 300 mL xylene are added into a reactor free of moisture and oxygen to form a polymerization solution. All chemicals are mixed through mechanical stirring and the solution is heated to 90° C. Then 562 mg stannous octoate is added into the polymerization solution and the solution is stirred at 120° C for 72 h to form long PLLA blocks.
Step 2: 20g GA, 20g TMC, 80mL xylene and 140mg stannous octoate are then added into the reactor to form short P(GA-co-TMC) blocks.
Step 3: Approximate 72 h later, the final product is precipitated into methanol, and dried in a vacuum oven at 95° C for 48 h or till constant weight.

### Example 4

Example 4 illustrates bioabsorbable stent preparation from the various copolymers that can be synthesized as described in Examples 1, 2a-b, and 3a-b.
Step 1: Extrude synthesized copolymer with long PLLA block and short fast eroding elastic block into tubing with designated ID and OD from a signal screw extruder at 200° C.
Step 2: Expand the extruded tubing to improve its mechanical property along the axial and radial directions.
Step 3: Cut stents from expanded tubings by femto second laser and sterilize all stents after crimping.

## Claims

1. An implantable medical device completely made of a biodegradable polymeric material, the biodegradable polymeric material including a block copolymer having a continuous phase block that is immiscible with a discrete phase block, wherein
the continuous phase block has a molecular weight from 50 kg/mol to 1000 kg/mol;
the discrete phase block has a molecular weight from 1 kg/mol to 50 kg/mol, and
the discrete phase blocks form discrete regions which are dispersed within a continuous phase composed by the continuous phase blocks.

2. The device of Claim 1, wherein the implantable medical device is a stent.

3. The device of Claim 1, wherein the block copolymers comprises a di-block copolymer, a first end of the di-block copolymer comprising a discrete phase block and a second end of the di-block copolymer comprising a continuous phase block,

4. The device of Claim 1, wherein the block copolymer comprises a tri-block copolymer, wherein the tri-block copolymer comprises a discrete phase block between two continuous phase blocks.

5. The device of Claim 1, wherein the block copolymer comprises a tri-block copolymer, wherein the tri-block copolymer oomprises a continuous phase block between two discrete phase blocks.

6. The device of Claim 1, wherein the block copolymer comprises a star-block copolymer having at least three arms, the arms having inner segments and outer segments, the inner segments comprising discrete phase blocks and the outer segments comprising continuous phase blocks.

7. The device of Claim 1, wherein the block copolymer comprises a star-block copolymer having at least three arms, the arms having inner segments and outer segments, the inner segments comprising continuous phase blocks and the outer segments comprising discrete phase blocks.

8. The device of Claim 1, wherein ratio of the molecular weight of the continuous phase blocks to the discrete phase blocks is 2:1 to 100:1.

9. The device of Claim 1, wherein the discrete phase blocks and the continuous phase blocks are biodegradable polymers.

10. The device of Claim 1, wherein the discrete phase blocks arc faster eroding than the continuous phase blocks.

11. The device of Claim 1, wherein the discrete phase blocks have a Tg below body temperature and the continuous phase blocks have a Tg above body temperature.

12. The device of Claim 1, wherein the discrete phase blocks are elastic and the continuous phase blocks are glassy at physiological conditions.

13. The device of Claim 1, wherein the continuous phase block is PLLA..

14. The device of Claim 1, wherein the discrete phase block is selected from the group consisting of P(GA-co-CL), P(GA-co-TMC), P(CL-co-TMC) and P(GA-co-CL-co-TMC).

## Patentansprüche

1. Eine implantierbare medizinische Vorrichtung, die vollständig aus einem biologisch abbaubaren polymeren Material besteht, wobei das biologisch abbaubare polymere Material ein Bockpolymer einschließt, das einen kontinuerlichen Phasenblock hat, welcher mit einem diskreten Phasenblock nicht mischbar ist, wobei
der kontinuerliche Phasenblock ein Molekulargewicht von 50 kg/mol bis 1000 kg/mol hat;
der diskrete Phasenblock ein Molekulargewicht von 1 kg/mol bis 50 kg/mol hat, und
die diskreten Phasenblöcke abgetrennte Regionen bilden, die innerhalb einer kontinuerliche phase bestehend aus den kontinuerlichen Phasenblöcken dispergiert sind.

2. Die Vorrichtung des Anspruchs 1, wobei die implantierbare medizinische Vorrichtung ein Stent ist.

3. Die Vorrichtung des Anspruchs 1, wobei das Blockcopolymer ein Diblockcopolymer umfasst, ein erstes Ende des Diblockcopolymers umfassend einen diskreten Phasenblock und ein zweites Ende des Diblockcopolymers umfassend einen kontinuerlichen Phasenblock.

4. Die Vorrichtung des Anspruchs 1, wobei das Blockcopolymer ein Triblockcopolymer umfasst, wobei das Triblockcopolymer einen diskreten Phasenblock zwischen zwei kontinuerlichen Phasenblöcken umfasst.

5. Die Vorrichtung des Anspruchs 1, wobei das Blockcopolymer ein Triblockcopolymer umfasst, wobei das Triblockcopolymer einen kontinuerlichen Phasenblock zwischen zwei diskreten Phasenblöcken umfasst.

6. Die Vorrichtung des Anspruchs 1, wobei das Blockcopolymer ein Sternblockcopolymer umfasst, das mindestens drei Arme hat, wobei die Arme innere Teile und äußere Teile haben, wobei die inneren Teile diskrete Phasenblöcke umfassen und die äußeren Teile kontinuerliche Phasenblöcke umfassen.

7. Die Vorrichtung des Anspruchs 1, wobei das Blockcopolymer ein Sternblockcopolymer umfasst, das mindestens drei Arme hat, wobei die Arme innere Teile und äußere Teile haben, wobei die inneren Teile kontinuerliche Phasenblöcke umfassen und die äußeren Teile diskrete Phasenblöcke umfassen.

8. Die Vorrichtung des Anspruchs 1, wobei das Verhältnis vom Molekulargewicht der kontinuerlichen Phasenblöcke zum Molekulargewicht der diskreten Phasenblöcke von 2:1 bis 100:1 reicht.

9. Die Vorrichtung des Anspruchs 1, wobei die diskreten Phasenblöcke und die kontinuerlichen Phasenblöcke biologisch abbaubare Polymere sind.

10. Die Vorrichtung des Anspruchs 1, wobei die diskreten Phasenblöcke schneller als die kontinuerlichen Phasenblöcke erodieren.

11. Die Vorrichtung des Anspruchs 1, wobei die diskreten Phasenblöcke eine Tg unterhalb der Körpertemperatur haben und die kontinuerlichen Phasenblöcke eine Tg oberhalb der Körpertemperatur haben.

12. Die Vorrichtung des Anspruchs 1, wobei die diskreten Phasenblöcke elastisch sind und die kontinuerlichen Phasenblöcke glasartig unter physiologischen Bedingungen sind.

13. Die Vorrichtung des Anspruchs 1, wobei der kontinuerlichen Phasenblock PLLA ist.

14. Die Vorrichtung des Anspruchs 1, wobei der diskreten Phasenblock ausgewählt aus der Gruppe bestehend aus P(GA-co-CL), P(GA-co-TMC), P(CL-co-TMC) und P(GA-co-CL-co-TMC) ist.

## Revendications

1. Un dispositif médical implantable entièrement fabriqué en un matériau polymère biodégradable, incluant le matériau polymère biodégradable un copolymère bloc ayant un bloc de phase continue qui n'est pas miscible avec un bloc de phase discrète, dans lequel
le bloc de phase continue a un poids moléculaire allant de 50 kg/mol à 1000 kg/mol ;
le bloc de phase discrète a un poids moléculaire allant de 1 kg/mol à 50 kg/mol ;
et
les blocs de phase discrète forment des régions discrètes qui sont dispersées dans une phase continue composée des bloques de phase continue.

2. Le dispositif de la revendication 1, dans lequel le dispositif médical implantable est un stent.

3. Le dispositif de la revendication 1, dans lequel le copolymère bloc comprend un copolymère dibloc, une première extrémité du copolymère dibloc comprenant un bloc de phase discrète et une deuxième extrémité du copolymère dibloc comprenant un bloc de phase continue.

4. Le dispositif de la revendication 1, dans lequel le copolymère bloc comprend un copolymère tribloc, dans lequel le copoylmère tribloc comprend un bloc de phase discrète entre deux blocs de phase continue.

5. Le dispositif de la revendication 1, dans lequel le copolymère bloc comprend un copolymère tribloc, dans lequel le copolymère tribloc comprend un bloc de phase continue entre deux blocs de phase discrète.

6. Le dispositif de la revendication 1, dans lequel le copolymère bloc comprend un copolymère bloc étoilé ayant au moins trois bras, ayant les bras des segments intérieurs et des segments extérieurs, comprenant les segments intérieurs des blocs de phase discrète et comprenant les segments extérieurs des blocs de phase continue.

7. Le dispositif de la revendication 1, dans lequel le copolymère bloc comprend un copolymère bloc étoilé ayant au moins trois bras, ayant les bras des segments intérieures et des segments extérieures, comprenant les segments intérieures des blocs de phase continue et comprenant les segments extérieures des blocs de phase discrète.

8. Le dispositif de la revendication 1, dans lequel le rapport du poids moléculaire des blocs de phase continue au poids moléculaire des blocs de phase discrète va de 2:1 à 100:1.

9. Le dispositif de la revendication 1, dans lequel les blocs de phase discrète et les blocs de phase continue sont des polymères biodégradables.

10. Le dispositif de la revendication 1, dans lequel les blocs de phase discrète sont érodés plus rapidement que les blocs de phase continue.

11. Le dispositif de la revendication 1, dans lequel les blocs de phase discrète ont une Tg inférieure à la température du corps et les blocs de phase continue ont une Tg supérieure à la température du corps.

12. Le dispositif de la revendication 1, dans lequel les blocs de phase discrète sont élastiques et les blocs de phase continue sont vitreux sous des conditions physiologiques.

13. Le dispositif de la revendication 1, dans lequel le bloc de phase continue est le PLLA.

14. Le dispositif de la revendication 1, dans lequel le bloc de phase discrète est choisi dans le groupe constitué du P(GA-co-CL), du P(GA-co-TMC), du P(CL-co-TMC) et du P(GA-co-CL-co-TMC).
